# EUROPÄISCHE PATENTSCHRIFT

(11) **EP 1 677 835 B1**
(45) Veröffentlichungstag und Bekanntmachung des Hinweises auf die Patenterteilung: **27.01.2010**
(21) Anmeldenummer: 04789964.6
(22) Anmeldetag: 12.10.2004
(51) Int. Cl.: A61L 2/14, C23C 14/56, F04B 7/00, F16K 11/076

(54) **VERFAHREN UND VORRICHTUNG ZUR VAKUUMVERSORGUNG MINDESTENS EINER BEARBEITUNGSSTATION FÜR EIN WERKSTÜCK**
METHOD AND DEVICE FOR SUPPLYING A VACUUM TO AT LEAST ONE MACHINING STATION FOR A WORKPIECE
PROCEDE ET DISPOSITIF D'APPROVISIONNEMENT D'UN VACUUM D'AU MOINS UNE STATION DE TRAITEMENT D'UNE PIECE

(30) Priorität: 31.10.2003 DE 10351330
(43) Veröffentlichungstag der Anmeldung: 12.07.2006
(73) Patentinhaber: KHS Corpoplast GmbH & Co. KG, 20539 Hamburg (DE)
(72) Erfinder: SIEBELS, Sönke, 22307 Hamburg (DE)
(74) Vertreter: Klickow, Hans-Henning
(86) Internationale Anmeldenummer: PCT/DE2004/002258
(87) Internationale Veröffentlichungsnummer: WO 2005/044317

(56) Entgegenhaltungen:
- US-A- 619 615
- US-B1- 6 231 732
- US-B1- 6 488 889

## Beschreibung

Die Erfindung betrifft ein Verfahren zur Versorgung mindestens einer Bearbeitungsstation für ein Werkstück mit einem Unterdruck, bei dem mindestens ein erster Teil einer versorgungseinrichtung für den Unterdruck auf einem gemeinsam mit der Bearbeitungsstation rotierenden Tragelement bewegt und relativ zu einem feststehenden Teil der Versorgungseinrichtung beweglich geführt wird.

Die Erfindung betrifft darüber hinaus eine Vorrichtung zur Versorgung mindestens einer Bearbeitungsstation für ein Werkstück mit einem Unterdruck, die eine Versorgungseinrichtung für den Unterdruck aufweist, von der mindestens ein erster Teil gemeinsam mit der Bearbeitungsstation auf einem rotationsfähigen Tragelement angeordnet ist und bei der der erste Teil der Versorgungseinrichtung relativ zu einem feststehenden zweiten Teil der Versorgungseinrichtung beweglich geführt ist.

Verfahren und Vorrichtungen zur Versorgung von Bearbeitungsstationen mit einem Unterdruck sind in vielfältigen Ausführungsformen bekannt. Bei der blastechnischen Herstellung von Behältern werden beispielsweise an einem rotierenden Übergaberad angeordnete Saugtaschen verwendet, die über einen Drehverteiler an eine Unterdruckquelle angeschlossen sind und die einen Transport von aus einer Blasstation ausgegebener geblasener Behälter zu einer Ausgabestrecke durchführen.

Vergleichbare Verfahren und Vorrichtungen werden auch angewendet, um eine Bearbeitung von Werkstücken zu unterstützen, die in eine Bearbeitungsstation mit einer Unterdruckkammer eingesetzt werden. Derartige Bearbeitungsverfahren können beispielsweise als Plasmasterilisation oder als Plasmabeschichtung realisiert werden. Die entsprechenden Bearbeitungen der Behälter können sich dabei auf die Behandlung von Innenoberflächen und/oder äußeren Oberflächen der Behälter beziehen.

Aus der PCT-WO 01/03186 ist es beispielsweise im Zusammenhang mit der Plasmabeschichtung von flaschenförmigen Werkstücken bekannt, eine gruppenweise Zuordnung unterschiedlicher Unterdruckquellen zu einer Mehrzahl von Bearbeitungsstationen vorzunehmen und die Steuerung der Unterdruckzufuhr unter Verwendung einer scheibenartigen Verteileinrichtung durchzuführen, bei der ein oberen Scheibenteil relativ zu einem feststehenden unteren Scheibenteil rotiert.

Aus der EP-A 0 943 699 ist es im Zusammenhang mit einer Schleuseneinrichtung bekannt, eine trommelartige Unterdruckverteilung zu realisieren, bei der ein Innenteil feststehend angeordnet ist und ein äußerer Trommelteil relativ zum Innenteil rotiert.

Aus der US-B1-6,231,732 ist bereits eine Vorrichtung zur Beschichtung von Halbleitersubstraten bekannt, bei der von stationär angeordneten inneren und äußeren Ringelementen Bearbeitungsstationen bereitgestellt werden. Zwischen den Ringelementen ist ein Transportring geführt, der mit Ausnehmungen zur Aufnahme und zum Transport der Substrate versehen ist. Die Substrate werden vom Transportring taktweise den einzelnen Bearbeitungsstationen zugeführt. An den Bearbeitungsstationen befinden sich Stationäranschlüsse für Prozeßgase und Vakuumversorgungen. Zwischen dem Transportring und den inneren und äußeren Ringelementen erstreckt sich ein dünner Spalt, der die einzelnen Bearbeitungsstationen vakuumtechnisch miteinander verbindet, gegenüber einer Umgebung jedoch abgedichtet ist.

In der US-B1-6,488,889 werden auf einem rotierenden Tragelement angeordnete Bearbeitungsstationen zur Durchführung einer Vakuumbeschichtung beschrieben. Eine Versorgung der Bearbeitungsstation mit Prozeßgasen und Unterdruck erfolgt über abgedichtete Ringverteiler.

Eine generelle Forderung im bekannten Stand der Technik besteht darin, eine möglichst hohe Dichtigkeit der Versorgungseinrichtung gegenüber einer Umgebung zu gewährleisten, um ein Eindringen von Umgebungsluft und eine hieraus resultierende Verminderung des Unterdruckes zu vermeiden. Derartige Maßnahmen zur Abdichtung bestehen beispielsweise in der Verwendung von federnd gelagerten Dichtungen, an denen das bewegliche Teil entlanggleitet oder die gemeinsam mit dem beweglichen Teil entlang des feststehenden Teils geführt sind. Gemäß einer anderen Variante ist es auch bekannt, zwischen dem feststehenden Teil und dem beweglichen Teil eine Fettschicht anzuordnen und hierdurch ein Eindringen von Umgebungsluft zu vermeiden.

Die bekannten Verfahren und Vorrichtungen führen aufgrund des Gleitens der Materialien aufeinander zu einem Verschleiß, der nur relativ geringe Standzeiten zuläßt. Als problematisch erweist es sich insbesondere, daß mit zunehmendem Verschleiß zeitlich veränderliche Leckagen auftreten, die nur sehr aufwendig kompensiert werden können.

Aufgabe der vorliegenden Erfindung ist es, ein Verfahren der einleitend genannten Art derart anzugeben, daß ein verminderter Verschleiß sowie eine erhöhte Prozeßstabilität erreicht werden.

Diese Aufgabe wird erfindungsgemäß dadurch gelöst, daß der erste Teil der Versorgungseinrichtung relativ zum zweiten Teil mit einem spaltartigen Abstand geführt wird, der mindestens bereichsweise mit einer Umgebung in Verbindung steht.

Weitere Aufgabe der vorliegenden Erfindung ist es, eine Vorrichtung der einleitend genannten Art derart zu konstruieren, daß eine verlängerte Lebensdauer erreicht wird.

Diese Aufgabe wird erfindungsgemäß dadurch gelöst, daß sich mindestens bereichsweise zwischen dem ersten Teil und dem zweiten Teil der versorgungseinrichtung ein Spalt erstreckt, der mindestens bereichsweise mit einer Umgebung verbunden ist.

Durch die Verwendung eines mindestens bereichsweise abdichtungsfreien Spaltes zwischen dem ersten Teil und dem zweiten Teil der Versorgungseinrichtung wird ein zum Stand der Technik konträrer Lösungsweg gegangen. Die bislang realisierten Verfahren und Konstruktionen haben nicht in ausreichender Weise berücksichtigt, daß bei der Erzeugung eines Vakuums relativ zu einer Umgebung eine maximale Druckdifferenz von 1 bar vorliegt. Darüber hinaus wurde nicht berücksichtigt, daß unabhängig von der jeweils realisierten Druckdifferenz die sich einstellende Luftströmung maximal eine Ausbreitungsgeschwindigkeit besitzen kann, die der Schallgeschwindigkeit entspricht. Dies hat zur Folge, daß für eine definierte und im wesentlichen konstant bleibende Spaltfläche eine maximale Strömungsmenge ermittelt werden kann, die durch eine erhöhte Pumpleistung kompensierbar ist.

Bei einer technisch realisierbaren minimalen Spaltbreite im Bereich weniger zehntel Millimeter ergibt sich eine zusätzlich erforderte Pumpleistung im Bereich von etwa 10 % bis 20 %, typischer Weise etwa 15 %, sowie hiermit im Zusammenhang stehende erhöhte Betriebskosten. Diesen erhöhten Betriebskosten stehen die Vermeidung häufiger Auswechslungen von Verschleißteilen sowie die Vermeidung hieraus resultierender Stillstandszeiten gegenüber. Bereits die Gegenrechnung der hiermit hervorgerufenen Materialeinsparung sowie die Vermeidung der Produktionsausfallzeiten führt insgesamt zu einer Kostenreduzierung. Darüber hinaus ist die auftretende Lekkage im wesentlichen zeitlich konstant, so daß eine relativ wenig aufwendige Kompensation möglich ist. Es kann somit zusätzlich zur wirtschaftlichen Einsparung eine deutlich verbesserte Prozeßqualität erreicht werden.

Eine typische Dimensionierung besteht darin, daß der Spalt eine Spaltbreite von etwa 0,1 mm bis 0,3 mm aufweist.

Zur Durchführung einer effektiven Druckabsenkung wird vorgeschlagen, daß mindestens zwei Unterdruckpumpen in einer Bewegungsrichtung des Tragelementes hintereinander angeordnet sind.

Insbesondere erweist es sich als vorteilhaft, daß mindestens zwei Unterdruckpumpen mit unterschiedlichen Unterdruckniveaus verwendet sind.

Eine hohe Produktionsrate kann dadurch erreicht werden, daß mindestens zwei Bearbeitungsstationen in Bewegungsrichtung des Tragelementes hintereinander angeordnet sind.

Eine Eingabe und Ausgabe der Werkstücke im Bereich der Bearbeitungsstation wird dadurch unterstützt, daß mindestens eine der Unterdruckpumpen auf dem rotierenden Tragelement angeordnet ist.

Gemäß einer anderen Konstruktionsvariante ist auch daran gedacht, daß mindestens eine der Bearbeitungsstationen auf dem rotierenden Tragelement angeordnet ist.

Eine kupplungsartige Ausbildung der Versorgungseinrichtung und/oder die Durchführung einer gesteuerten Verteilung von Unterdruck wird dadurch unterstützt, daß die Versorgungseinrichtung mindestens bereichsweise zwei Scheiben aufweist, zwischen den sich der Spalt erstreckt.

Gemäß einer anderen Ausführungsform ist es auch möglich, daß die Versorgungseinrichtung mindestens ein zylinderartiges Innenteil sowie ein das Innenteil umgebendes Außenteil umfaßt, zwischen denen sich der Spalt erstreckt.

Bei Verwendung einer Mehrzahl von Bearbeitungsstationen erweist es sich als zweckmäßig, daß die Versorgungseinrichtung mindestens bereichsweise als ein Unterdruckverteiler ausgebildet ist.

Weitere Steuermöglichkeiten zur Vorgabe der sich einstellenden Unterdrücke werden dadurch bereitgestellt, daß die Bearbeitungsstation mindestens ein Steuerventil zur vorgebbaren Trennung zweier Innenraumbereiche der Bearbeitungsstation umfaßt.

Ein Anwendungsbeispiel wird dadurch definiert, daß die Bearbeitungsstation Saugtaschen zur Durchführung einer Übergabe der Werkstücke umfaßt.

Darüber hinaus ist auch daran gedacht, daß die Bearbeitungsstation zur Plasmabehandlung von Werkstücken ausgebildet ist.

Zur Unterstützung vorgegebener Prozeßbedingungen im Bereich der Bearbeitungsstation ist daran gedacht, daß mindestens bereichsweise entlang des Spaltes durch mindestens zwei Unterdruckpumpen ein Druckprofil bereitgestellt ist.

Bei einer innenliegenden Anordnung des rotationsfähigen Tragelementes ist vorgesehen, daß das Tragelement als ein Tragrad ausgebildet ist.

Bei einer außenliegenden Anordnung des rotierenden Tragelementes ist es zweckmäßig, daß das Tragelement als ein Tragring ausgebildet ist.

In den Zeichnungen sind Ausführungsbeispiele der Erfindung schematisch dargestellt. Es zeigen:
- Fig. 1: eine teilweise Darstellung einer schemati- schen Draufsicht auf ein Tragrad mit Un- terdruckpumpen sowie stationär entlang ei- nes Umfanges des Tragrades angeordneter Bearbeitungsstationen,
- Fig. 2: eine schematische Darstellung einer Bear- beitungsstation zur Behandlung von fla- schenförmigen Werkstücken,
- Fig. 3: ein Volumenfluß-Zeit-Diagramm zur Veran- schaulichung einer geblockten Strömung,
- Fig. 4: eine schematische Darstellung zur Veran- schaulichung der Verwendung zusätzlicher Dichtelemente und
- Fig. 5: eine vollständige schematische Darstellung des in Fig. 1 lediglich bereichsweise dar- gestellten Tragrades mit Unterdruckpumpen.

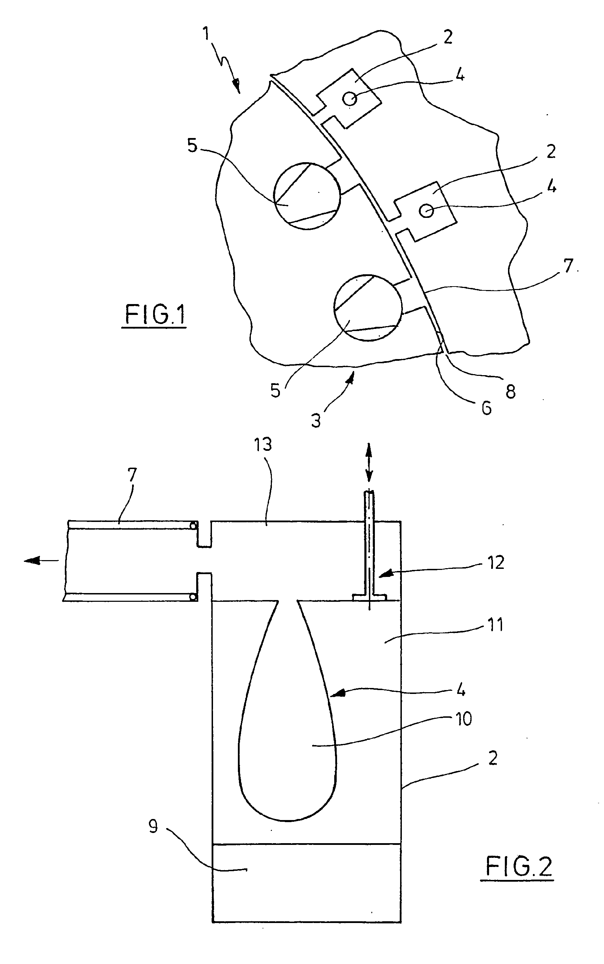

Gemäß der Darstellung in Fig. 1 ist eine Versorgungseinrichtung (1) zur Versorgung von Bearbeitungsstationen (2) mit Unterdruck mit einem Tragelement (3) versehen, daß rotationsfähig gelagert und al ein Tragrad ausgebildet ist. Im Bereich der Bearbeitungsstation (2) sind Werkstücke (4) anordbar und auf dem Tragelement (3) sind Unterdruckpumpen (5) angeordnet

Zum Anschluß der Bearbeitungsstation (2) an die Unterdruckpumpen (5) ist im Bereich des Tragelementes (3) ein erster Teil der Versorgungseinrichtung (1) angeordnet und im Bereich der Bearbeitungsstation (2) erstreckt sich zu diesem zweck ein zweiter Teil (7) der versorgungseinrichtung (1). Zwischen dem ersten Teil (6) und dem zweiten (7) der Versorgungseinrichtung (1) ist ein Spalt (8) angeordnet, der sich mindestens bereichsweise abdichtungsfrei erstreckt.

Die dargestellten Ausgangsstutzen decken im wesentlichen die gesamte Höhe bzw. Breite des Spaltes (8) ab, um eine definierte Trennung der Druckbereiche sicherzustellen.

Fig. 2 veranschaulicht schematisch den Aufbau einer Bearbeitungsstation für Werkstücke (4). Bei einer Ausbildung der Bearbeitungsstation (2) zur Plasmabehandlung von Werkstücken (4) ist die Bearbeitungsstation (2) typischerweise mit einer Mikrowelle (9) ausgestattet. Gemäß der Ausführungsform in Fig. 2 ist das Werkstück (4) als ein Hohlkörper ausgebildet und ein Innenraum (10) des Werkstückes (4) sowie ein das Werkstück (4) bereichsweise umgebender Kammerraum (11) der Bearbeitungsstation (2) können mit voneinander abweichenden Unterdrücken versehen werden.

Die unterschiedlichen Unterdrücke werden hierbei derart erzeugt, daß im Innenraum des Werkstückes (4) ein geringerer Druck als im Kammerraum (11) bereitgestellt wird. Dies wird gerätetechnisch dadurch realisiert, daß während des Abpumpvorganges nach Erreichen des vorgegebenen Unterdruckes im Bereich des Kammerraumes (11) ein Steuerventil (12) schließt, das den Kammerraum (11) mit einer Vorkammer (13) verbindet, in die das zweite Teil (7) der Versorgungsrichtung (1) sowie der Innenraum (10) des Werkstückes (4) einmünden. Bei einer fortgesetzten Durchführung der Abpumpvorgänge werden nach einem Schließen des Steuerventils (12) nur noch der Bereich der Vorkammer (13) sowie der Innenraum (10) hinsichtlich ihres Druckniveaus weiter abgesenkt, der Unterdruck im Bereich des Kammerraumes (11) bleibt hingegen im wesentlichen unverändert.

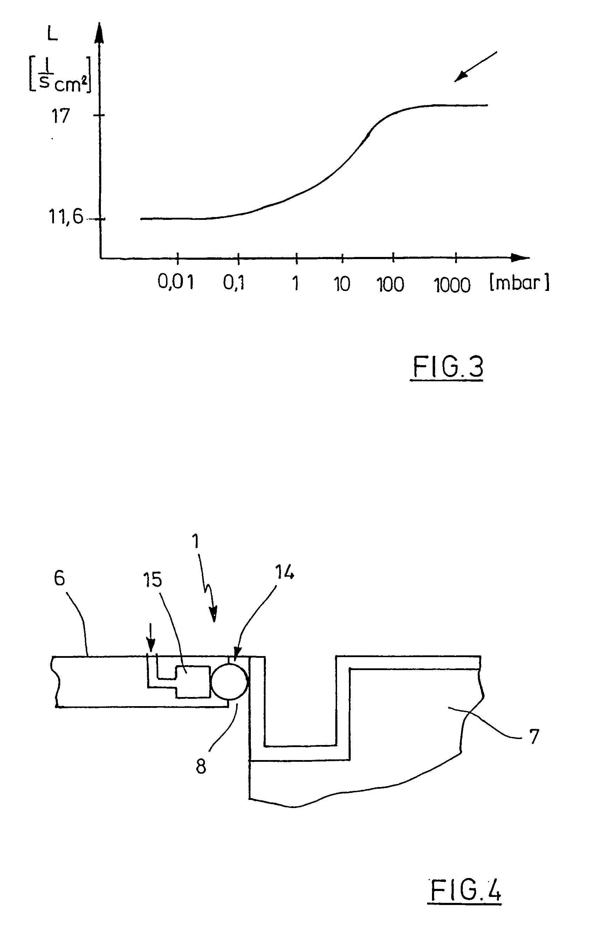

Fig. 3 veranschaulicht hinsichtlich eines spaltförmigen Strömungskanals die Abhängigkeit des Strömungsflusses in Abhängigkeit vom Druck. Es ist zu erkennen, daß zunächst mit einer zunehmenden Druckdifferenz am Spalt (8) der Volumenfluß zunimmt. In Abhängigkeit von der Spaltgeometrie stellt sich aber ab einer bestimmten Sättigung eine sogenannte geblockte Strömung ein, die daraus resultiert, daß strömende Luft sich maximal mit Schallgeschwindigkeit ausbreiten kann. Auch eine zunehmende Druckdifferenz führt im Bereich dieser geblockten Strömung nicht zu einer nennenswerten Zunahme des Strömungsflusses.

Fig. 4 zeigt eine Ausführungsform, bei der zusätzlich in einem Bereich des Spaltes (8) ein Dichtelement (14) verwendet wird. Das Dichtelement (14) ist von einer pneumatischen Stelleinrichtung (15) verspannbar, so daß die hier einwirkenden Dichtkräfte vorgebbar sind. Darüber hinaus ist es auch möglich, über das pneumatische Stellelement (15) zeitlich veränderliche Abdichtungskräfte vorzugeben, um eine hohe Abdichtung und einen hieraus resultierenden Verschleiß lediglich dann vorzugeben, wenn die jeweiligen Prozeßbedingungen dies auch erfordern. Es können beispielsweise zwei umlaufende Dichtringe als Dichtelement (14) verwendet werden.

Eine konstruktive Realisierung des Spaltes (8) derart, daß dieser bereichsweise gegenüber einer Umgebung offen sowie bereichsweise abgedichtet ist, führt zu einer Optimierung der Eigenschaften und trägt zu einer Realisierung einer optimalen Kompromisses zwischen entstehendem Verschleiß sowie optimaler Abdichtung bei.

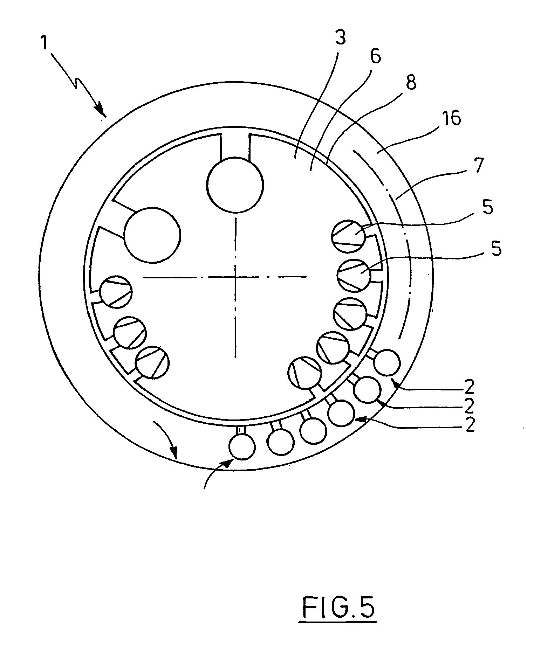

Fig. 5 zeigt eine vollständige Darstellung des in Fig. 1 lediglich teilweise dargestellten Tragelementes (3). Alternativ zur Verwendung eines rotierenden Tragrades ist es ebenfalls möglich, die in Fig. 5 dargestellten Unterdruckpumpen (5) auf einem stationären Mittelteil anzuordnen und die Bearbeitungsstation (3) auf einem rotationsfähigen Tragring zu positionieren. Ebenfalls ist es denkbar, entgegengesetzt zur Darstellung in Fig. 5 die Unterdruckpumpen (5) außen liegend und die Bearbeitungsstationen (2) innenliegend anzuordnen. Auch hier kann in Abhängigkeit von den konstruktiven Randbedingungen vorgegeben werden, ob die innenliegenden oder die außenliegenden Bauelemente auf einem rotationsfähigen Tragelement (3) angeordnet werden.

Gemäß Fig. 5 sind im Bereich von einer Ausgabe der Werkstücke (4) aus den Bearbeitungsstationen (2) eine Mehrzahl von Unterdruckpumpen (5) in Transportrichtung hintereinander angeordnet, die einzelne Druckstufen bereitstellen, um ein Überströmen von Gas in den Prozeßraum zu verhindern.

## Patentansprüche

1. Verfahren zur Versorgung mindestens einer Bearbeitungsstation (2) für ein Werkstück mit einem Unterdruck, bei dem mindestens ein erster Teil (6) einer Versorgungseinrichtung (1) für den Unterdruck gemeinsam mit der Bearbeitungsstation auf einem rotierenden Tragelement (3) bewegt und relativ zu einem feststehenden Teil der Versorgungseinrichtung (1) beweglich geführt wird, **dadurch gekennzeichnet, daß** der erste Teil (6) der Versorgungseinrichtung (1) relativ zum zweiten Teil (7) mit einem spaltartigen Abstand geführt wird, der mindestens bereichsweise mit einer Umgebung in Verbindung steht.

2. Verfahren nach Anspruch 1, **dadurch gekennzeichnet, daß** der Unterdruck durch mindestens zwei Unterdruckpumpen (5) mit unterschiedlichen Unterdruckniveaus erzeugt wird.

3. Verfahren nach Anspruch 1 oder 2, **dadurch gekennzeichnet, daß** mindestens zwei Unterdruckpumpen (5) in Richtung einer Bewegung des rotierenden Tragelementes (3) hintereinander positioniert werden.

4. Verfahren nach einem der Ansprüche 1 bis 3, **dadurch gekennzeichnet, daß** mindestens zwei Bearbeitungsstationen (2) in eine Bewegungsrichtung des rotierenden Tragelementes (3) hintereinander positioniert werden.

5. Verfahren nach einem der Ansprüche 1 bis 4, **dadurch gekennzeichnet, daß** die Bearbeitungsstation (2) gemeinsam mit dem Tragelement (3) bewegt wird.

6. Verfahren nach einem der Ansprüche 1 bis 4, **dadurch gekennzeichnet, daß** die Unterdruckpumpe (5) gemeinsam mit dem Tragelement (3) bewegt wird.

7. Verfahren nach einem der Anspräche 1 bis 6, **dadurch gekennzeichnet, daß** die Versorgungseinrichtung (1) eine Verteilung des Unterdrukkes auf eine Mehrzahl von Bearbeitungsstationen (2) durchführt.

8. Verfahren nach einem der Ansprüche 1 bis 7, **dadurch gekennzeichnet, daß** die Bearbeitungsstation (2) eine Plasmabehandlung der Werkstücke (4) durchführt.

9. Vorrichtung zur Versorgung mindestens einer Bearbeitungsstation (2) für ein Werkstück mit einem Unterdruck, die eine Versorgungseinrichtung (1) für den Unterdruck aufweist, von der mindestens ein erster Teil (6) gemeinsam mit der Bearbeitungsstation auf einem rotationsfähigen Tragelement (3) angeordnet ist und bei der der erste Teil (6) der Versorgungseinrichtung (1) relativ zu einem feststehenden zweiten Teil (7) der Versorgungseinrichtung (1) beweglich geführt ist, **dadurch gekennzeichnet, daß** sich mindestens bereichsweise zwischen dem ersten Teil (6) und dem zweiten Teil (7) der Versorgungseinrichtung (1) ein Spalt (8) erstreckt, der mindestens bereichsweise mit einer Umgebung verbunden ist.

10. Vorrichtung nach Anspruch 9, **dadurch gekennzeichnet, daß** der Spalt (8) eine Spaltbreite von etwa 0,1 mm bis 0,3 mm aufweist.

11. Vorrichtung nach Anspruch 9 oder 10, **dadurch gekennzeichnet, daß** mindestens zwei Unterdruckpumpen (5) in einer Bewegungsrichtung des Tragelementes (3) hintereinander angeordnet sind.

12. Vorrichtung nach einem der Ansprüche 9 bis 11, **dadurch gekennzeichnet, daß** mindestens zwei Unterdruckpumpen (5) mit unterschiedlichen Unterdruckniveaus verwendet sind.

13. Vorrichtung nach einem der Ansprüche 9 bis 12, **dadurch gekennzeichnet, daß** mindestens zwei Bearbeitungsstationen (2) in Bewegungsrichtung des Tragelementes (3) hintereinander angeordnet sind.

14. Vorrichtung nach einem der Ansprüche 9 bis 13, **dadurch gekennzeichnet, daß** mindestens eine der Unterdruckpumpen (5) auf dem rotierenden Tragelement (3) angeordnet ist.

15. Vorrichtung nach einem der Ansprüche 9 bis 13, **dadurch gekennzeichnet, daß** mindestens eine der Bearbeitungsstationen (2) auf dem rotierenden Tragelement (3) angeordnet ist.

16. Vorrichtung nach einem der Ansprüche 11 bis 15, **dadurch gekennzeichnet, daß** die Versorgungseinrichtung (1) mindestens bereichsweise zwei Scheiben aufweist, zwischen den sich der Spalt (8) erstreckt.

17. Vorrichtung nach einem der Ansprüche 9 bis 15, **dadurch gekennzeichnet, daß** die Versorgungseinrichtung (1) mindestens ein zylinderartiges Innenteil sowie ein das Innenteil umgebendes Außenteil umfaßt, zwischen denen sich der Spalt (8) erstreckt.

18. Vorrichtung nach einem der Ansprüche 9 bis 17, **dadurch gekennzeichnet, daß** die Versorgungseinrichtung (1) mindestens bereichsweise als ein Unterdruckverteiler ausgebildet ist.

19. Vorrichtung nach einem der Ansprüche 9 bis 18, **dadurch gekennzeichnet, daß** die Bearbeitungsstation (2) mindestens ein Steuerventil (12) zur vorgebbaren Trennung zweier Innenraumbereiche der Bearbeitungsstation (2) umfaßt.

20. Vorrichtung nach einem der Anspruche 9 bis 19, **dadurch gekennzeichnet, daß** die Bearbeitungsstation (2) Saugtaschen zur Durchführung einer Übergabe der Werkstücke (4) umfaßt.

21. Vorrichtung nach einem der Ansprüche 9 bis 19, **dadurch gekennzeichnet, daß** die Bearbeitungsstation (2) zur Plasmabehandlung von Werkstücken (4) ausgebildet ist.

22. Vorrichtung nach einem der Ansprüche 9 bis 21, **dadurch gekennzeichnet, daß** mindestens bereichsweise entlang des Spaltes (8) durch mindestens zwei Unterdruckpumpen (5) ein Druckprofil bereitgestellt ist.

23. Vorrichtung nach einem der Ansprüche 9 bis 22, **dadurch gekennzeichnet, daß** das Tragelement (3) als ein Tragrad ausgebildet ist.

24. Vorrichtung nach einem der Ansprüche 9 bis 22, **dadurch gekennzeichnet, daß** das Tragelement (3) als ein Tragring ausgebildet ist.

## Claims

1. Method for supplying a reduced pressure to at least one processing station (2) for a workpiece, wherein at least a first component (6) of a supply device (1) for the reduced pressure moves together with the processing station on a rotating carrier element (3) and is guided so as to be able to be moved relative to a stationary component of the supply device (1), **characterised in that** the first component (6) of the supply device (1) is guided relative to the second component (7) with a gap-like spacing which is at least partially connected to an environment.

2. Method according to claim 1, **characterised in that** the reduced pressure is produced by means of at least two vacuum pumps (5) with different reduced pressure levels.

3. Method according to claim 1 or claim 2, **characterised in that** at least two vacuum pumps (5) are positioned one behind the other in the movement direction of the rotating carrier element (3).

4. Method according to any one of claims 1 to 3, **characterised in that** at least two processing stations (2) are positioned one behind the other in a movement direction of the rotating carrier element (3).

5. Method according to any one of claims 1 to 4, **characterised in that** the processing station (2) is moved together with the carrier element (3).

6. Method according to any one of claims 1 to 4, **characterised in that** the vacuum pump (5) is moved together with the carrier element (3).

7. Method according to any one of claims 1 to 6, **characterised in that** the supply device (1) distributes the reduced pressure to a plurality of processing stations (2).

8. Method according to any one of claims 1 to 7, **characterised in that** the processing station (2) carries out a plasma treatment of the workpieces (4).

9. Device for supplying a reduced pressure to at least one processing station (2) for a workpiece, which has a supply device (1) for the reduced pressure, at least a first component (6) of which is arranged together with the processing station on a rotatable carrier element (3), and in which the first component (6) of the supply device (1) is guided so as to be able to be moved relative to a stationary second component (7) of the supply device (1), **characterised in that**, at least partially between the first component (6) and the second component (7) of the supply device (1), there extends a gap (8) which is at least partially connected to an environment.

10. Device according to claim 9, **characterised in that** the gap (8) has a gap width of approximately from 0.1 mm to 0.3 mm.

11. Device according to claim 9 or claim 10, **characterised in that** at least two vacuum pumps (5) are arranged one behind the other in a movement direction of the carrier element (3).

12. Device according to any one of claims 9 to 11, **characterised in that** at least two vacuum pumps (5) with different reduced pressure levels are used.

13. Device according to any one of claims 9 to 12, **characterised in that** at least two processing stations (2) are arranged one behind the other in the movement direction of the carrier element (3).

14. Device according to any one of claims 9 to 13, **characterised in that** at least one of the vacuum pumps (5) is arranged on the rotating carrier element (3).

15. Device according to any one of claims 9 to 13, **characterised in that** at least one of the processing stations (2) is arranged on the rotating carrier element (3).

16. Device according to any one of claims 11 to 15, **characterised in that** the supply device (1) at least partially has two plates, between which the gap (8) extends.

17. Device according to any one of claims 9 to 15, **characterised in that** the supply device (1) comprises at least one cylindrical inner portion and an outer portion which surrounds the inner portion, between which the gap (8) extends.

18. Device according to any one of claims 9 to 17, **characterised in that** the supply device (1) is constructed at least partially as a vacuum distributor.

19. Device according to any one of claims 9 to 18, **characterised in that** the processing station (2) comprises at least one control valve (12) for predeterminable separation of two internal space regions of the processing station (2).

20. Device according to any one of claims 9 to 19, **characterised in that** the processing station (2) comprises suction pockets for transferring the workpieces (4).

21. Device according to any one of claims 9 to 19, **characterised in that** the processing station (2) is constructed for plasma treatment of workpieces (4).

22. Device according to any one of claims 9 to 21, **characterised in that**, at least partially along the gap (8), a pressure profile is provided by at least two vacuum pumps (5).

23. Device according to any one of claims 9 to 22, **characterised in that** the carrier element (3) is constructed as a carrier wheel.

24. Device according to any one of claims 9 to 22, **characterised in that** the carrier element (3) is constructed as a carrier ring.

## Revendications

1. Procédé pour la génération d'un vide dans au moins une station de traitement (2) d'une pièce à usiner, dans lequel au moins une premières partie (6) d'un générateur de vide (1) est mue, en commun avec la station de traitement, sur un élément de support rotatif (3) et peut être guidée, mobile par rapport à une partie fixe du générateur de vide (1), **caractérisé en ce que** la première partie (6) du générateur de vide (1) est guidée, par rapport à la deuxième partie (7), avec un interstice qui est relié, au moins par sections, au milieu ambiant.

2. Procédé selon la revendication 1, **caractérisé en ce que** le vide est généré par au moins deux pompes à vide (5) avec des niveaux de vide différents.

3. Procédé selon la revendication 1 ou 2, **caractérisé en ce que** deux pompes à vide (5) au moins sont positionnées l'une derrière l'autre, dans la direction d'un mouvement de l'élément de support rotatif (3).

4. Procédé selon l'une des revendications 1 à 3, **caractérisé en ce que** deux stations de traitement (2) au moins sont positionnées, l'une derrière l'autre, dans la direction d'un mouvement de l'élément de support (3) rotatif.

5. Procédé selon l'une des revendications 1 à 4, **caractérisé en ce que** la station de traitement (2) est mue en commun avec l'élément de support (3).

6. Procédé selon l'une des revendications 1 à 4, **caractérisé en ce que** la pompe à vide (5) est mue en commun avec l'élément de support (3).

7. Procédé selon l'une des revendications 1 à 6, **caractérisé en ce que** le générateur de vide (1) effectue une répartition de vide sur plusieurs stations de traitement (2).

8. Procédé selon l'une des revendications 1 à 7, **caractérisé en ce que** la station de traitement (2) exécute un traitement au plasma des pièces (4).

9. Dispositif pour la génération d'un vide dans au moins une station de traitement (2) pour une pièce à usiner, qui présente un générateur de vide (1), dont une première partie (6) est disposée, en commun avec la station de traitement, sur un élément de support capable de tourner en rotation (3), et dans lequel la première partie (6) du générateur de vide (1) est déplacée par rapport à une deuxième partie du générateur de vide (1), **caractérisé en ce qu'**un interstice (8) s'étend, au moins par sections, entre la premières partie (6) et la deuxième partie (7), lequel interstice est relié, au moins par sections, au milieu ambiant.

10. Dispositif selon la revendication 9, **caractérisé en ce que** l'interstice (8) présente une largeur d'environ 0,1 mm à 0,3 mm.

11. Dispositif selon revendication 9 ou 10, **caractérisé en ce que** deux pompes à vide (5), au moins, sont disposées, l'une derrière l'autre, dans une direction de déplacement de l'élément de support (3).

12. Dispositif selon l'une des revendications 9 à 11, **caractérisé en ce que** deux pompes à vide (5), au moins, sont utilisées avec des niveaux de vide différents.

13. Dispositif selon l'une des revendications 9 à 12, **caractérisé en ce que** deux stations de traitement (2) au moins sont disposées dans la direction de déplacement de l'élément de support rotatif (3).

14. Dispositif selon l'une des revendications 9 à 13, **caractérisé en ce que** l'une des pompes à vide (5), au moins, est disposée sur l'élément de support rotatif (3).

15. Dispositif selon l'une des revendications 9 à 13, **caractérisé en ce que** l'une des stations de traitement (2), au moins, est disposée sur l'élément de support rotatif (3).

16. Dispositif selon l'une des revendications 11 à 15, **caractérisé en ce que** le générateur de vide (1) présente, au moins par sections, deux disques, entre lesquels s'étend l'interstice (8).

17. Dispositif selon l'une des revendications 9 à 15, **caractérisé en ce que** le générateur de vide (1) comprend au moins une partie intérieure, genre cylindre, ainsi qu'une partie extérieure, qui entoure la partie intérieure, entre lesquelles s'étend l'interstice (8).

18. Dispositif selon l'une des revendications 9 à 17, **caractérisé en ce que** le générateur de vide (1) est conçu, au moins par sections, en tant que distributeur de vide.

19. Dispositif selon l'une des revendications 9 à 18, **caractérisé en ce que** la station de traitement (2) comprend au moins une soupape de commande (12) pour la séparation prédéterminable de deux zones intérieures de la station de traitement (2).

20. Dispositif selon l'une des revendications 9 à 19, **caractérisé en ce que** la station de traitement (2) comprend des poches d'aspiration pour la réalisation d'un transfert des pièces (4).

21. Dispositif selon l'une des revendications 9 à 19, **caractérisé en ce que** la station de traitement (2) est conçue pour le traitement au plasma de pièces à usiner (4).

22. Dispositif selon l'une des revendications 9 à 21, **caractérisé en ce que**, le long de l'interstice (8), un profil de pression est produit, au moins par sections, par au moins deux pompes à vide (5).

23. Dispositif selon l'une des revendications 9 à 22, **caractérisé en ce que** l'élément de support (3) est réalisé sous la forme d'une roue de support.

24. Dispositif selon l'une des revendications 9 à 22, **caractérisé en ce que** l'élément de support (3) est réalisé sous la forme d'un anneau de support.
